# EUROPEAN PATENT SPECIFICATION

(11) **EP 3 510 870 B1**
(45) Date of publication and mention of the grant of the patent: **26.07.2023**
(21) Application number: 18382015.8
(22) Date of filing: 16.01.2018
(51) Int. Cl.: A22C 13/00, A23B 4/10, A23B 4/22, A23L 13/60, B65D 81/28

(54) **FOOD CASING WITH ANTIFUNGAL PROPERTIES AND METHOD FOR PRODUCTION THEREOF**
LEBENSMITTELHÜLLE MIT ANTIMYKOTISCHEN EIGENSCHAFTEN UND VERFAHREN ZUR HERSTELLUNG DAVON
ENVELOPPE ALIMENTAIRE AYANT DES PROPRIÉTÉS ANTIFONGIQUES ET SON PROCÉDÉ DE FABRICATION

(43) Date of publication of application: 17.07.2019
(73) Proprietor: VISCOFAN S.A., 31192 Tajonar, Navarra (ES)
(72) Inventor: DE LA FUENTE MELIDA, Clara, 31192 Tajonar, Navarra (ES); JAUREGUI ARBIZU, Blanca, 31192 Tajonar, Navarra (ES); POHL, Matthias, 31192 Tajonar, Navarra (ES)
(74) Representative: Herrero & Asociados, S.L.

(56) References cited:
- WO-A1-2005/018322
- US-A- 5 230 933
- US-A- 6 143 344
- US-A1- 2016 213 051

## Description

### TECHNICAL FIELD OF THE INVENTION

The present invention relates to the field of food casing with antifungal properties for the production of meat products such as sausages, especially dry sausages. More specifically, the present invention is related to a food casing with antifungal properties containing natamycin, which effect has been enhanced by modifying the availability of natamycin on the casing surface. The invention is also directed to the method for production of said food casing as well as to a meat product prepared by stuffing said food casing with meat or a meat emulsion.

### BACKGROUND OF THE INVENTION

Artificial food casings have been used for decades in the production of meat products. Fiber reinforced casing, known also as fibrous casing, is a tube of hemp paper covered with regenerated cellulose. This type of casing is commonly used for dry and semi-dry sausages, due to its water permeability and caliber control.

Dry sausages are popular products in Europe, especially in Eastern European and Mediterranean countries. The production of dry sausages involves three steps; first, the meat emulsion is stuffed into a fibrous casing; second, the meat ferments due to the action of the Lactic Acid Bacteria; and finally, the sausages dries for several weeks in curing or drying rooms. This process provides the sausage its unique organoleptic properties.

During the drying process, the ambient moisture should be kept relatively high so that the sausage does not get a dry crust prematurely. The high moisture environment, together with the warm temperatures of the drying rooms promotes the growth of fungus.

During the first part of the drying cycle, mold is more prone to growth. The warm temperatures (24°C), and the high humidity level (between 90-100% HR) encourages mold spores to adhere to the casing and growth.

During the second part of the cycle, the temperature drops down to 12°C, and the humidity is reduced to 70-80 %HR. Under these conditions, yeast may grow.

Mold and yeast may cause allergic reactions and respiratory problems. In addition, some species can produce mycotoxins, poisonous substances that may lead to serious diseases.

Molds spores can float through the air, and easily spread in the drying chambers. When the conditions are suitable, they may start the growth cycle again. Drying rooms of meat manufacturers present extremely challenging conditions to prevent fungi growth due to the constant air circulation, and the temperature and humidity levels.

When mold and/or yeast are detected on the surface of sausages, the fungus and its toxins have already invaded deeply the product. At that point, cleaning the surface of the sausage is not enough to assure the safety of the product.

In order to prevent fungi growth under these conditions, a potent antifungal or mixture of antifungals needs to be applied to the casing. International Food Regulations restrict the number of substances to be in contact with meat products and their limits. Substances have to be suitable for food contact, not only in their original form, but also after changes of pH, exposure to heat, moisture, etc. In addition, the antifungal compound or mixture should not inhibit the growth of Lactic Acid Bacteria that produces the meat fermentation.

Casing manufacturers have been trying to develop a casing with antifungal properties for decades. Several products have been described in the literature.

US 4867204 discloses a ready-to-use casing with an antifungal agent. The antifungal agent is preferably propylene glycol or the calcium, potassium or sodium salts of propionic, sorbic or benzoic acid. These preservatives have been thoroughly studied for decades, and it is well-known that they have a very limited antifungal effect. Very high concentration of antifungal agent is needed to prevent the average contamination of industrial drying chambers. However, reaching such required quantities imply other technological problems like deposits in machines, poor appearance of the casing, etc.

EP 0378069 relates to a food casing comprising a di-n-decyldimethylammonium compound (DDAC) alone or in combination with other antifungals like sorbic acid, glycerol monolaurate or isothiazolone. The problem with this solution is that current limit of DDAC that Food Regulations allows for meat products is not enough to produce a significant inhibition. Even in combination with other antifungals mentioned in the document, it is unlikely that inhibition of fungal growth under industrial conditions is achieved. Furthermore, DDAC is very soluble in water, which makes its use not safe for casings that are soaked in water prior to stuffing, since the concentration of DDAC will tend to increase in the soaking baths.

EP 1013173 teaches to a multilayer plastic food casing, which is optionally sprayed with a fungicide solution in one of its layers. The use of plastic casings is not suitable for dry sausages, since they are not water permeable, and will impede the drying process.

Another solution is provided in EP 2363024 which relates to a food casing comprising N-Lauroylarginate ethyl ester hydrochloride (LAECI) to prevent fungi and bacteria growth. However, the use of LAECI is not allowed by the European Food Regulations for dry sausages.

In turn, EP 2859796 relates to a method for producing a tubular food casing with biocidal attributes. The biocidal mixture comprises; a) a biocidal substance, b) a tryglyceride and c) a solubilizing agent in the form of a "micellated solubilizate", which makes the casing waterproof. The problem of this solution is that most of the antifungals mentioned have a strong smell and are only effective when used in large quantities. At this level, the organoleptic properties of the meat product will be modified by the antifungal. In addition, some of them are volatile and difficult to keep in the casing for long periods of time. It is believed that the smell and price of these compounds, along with other technological problems, made this solution not suitable for its marketing. To date, no presence of this product was found in the market.

A further antifungal food casing which includes a salt of pyrithione is also disclosed in EP 2944199. Pyrithione and its salts are not classified as food grade additives according to the European Regulations. In this sense, if the antifungal migrates from the casing, it would compromise the food safety of the sausage.

Natamycin is an antifungal agent that targets ergosterols of mold and yeast's membranes, producing eventually the lysis of the microorganism. The membranes of bacteria have no ergoesterols, so they remain unaffected. Natamycin has a strong antifungal effect, and no resistance has been reported after decades of use. For these reasons, it appears as a perfect candidate for mold and yeast inhibition on sausages and casing. Some attempts to use natamycin as antifungal agent in food products have been reported so far.

For instance, WO 01/80658 reports a biologically degradable coating for food and food ingredients, in particular for cheese, which comprises a) 3-35% of globular protein, b) 5-55% of sugar, c) 3-20% of fat, d) 5-15% plasticizer, e) 0-5% of thickener, f) 0-20% of filler and g) 0-5% of a fungicide, wherein the dry solids content is 20-60%. The fungicide reported in this document is preferably natamycin. The problem of this solution is that the presence of such a high amount of solids makes not suitable this coating for dry sausages since it impedes the drying process. Furthermore, most of the food grade coatings are meant to be applied by immersion, wherein high viscosities usually help with the adherence of the coating to the food. In the casing industry, high viscosity coatings present several technological difficulties to be applied using current winding technology.

US-A1-2016/0213051 discloses a method for producing a processed edible product comprising a complex of at least one antimicrobial compound and a polyelectrolyte complex of a polyanion and a polycation.

Although natamycin's antifungal properties are well-known, surprisingly, there is not any antimold casing in the market based on natamycin. This may be explained because natamycin present a major technological drawback that, to date, seems not to have been solved.

In order to inhibit fungi, natamycin has to be dissolved. The solid form of natamycin does not have antifungal properties. This means that the antifungal effect of natamycin is directly linked its solubility.

Furthermore, once it is solved, its availability to diffuse, and its stability in its solved form will define its antifungal effectivity.

Natamycin has almost negligible solubility in water, and decomposes by the light, oxygen, heat, extreme pH, and hydrolysis. In addition, its soluble form is much less stable than its crystals.

These properties make very challenging attaching natamycin to the casing in quantities large enough to prevent mold and yeast growth at sausages facilities, which are usually highly contaminated with mold and/or yeast.

Natamycin cannot be applied using the usual methods to incorporate additives to casings. In principle, the best approach to assure that natamycin is firmly fixed to the casing wall will be mixing the natamycin with the viscose, and then extrude the tube. Unfortunately, this is not a valid option. Right after the tube is extruded, it goes through a long chemical process of regeneration of the cellulose, which comprises extreme pH changes and heat. This process breaks and decomposes the natamycin.

Another common approach in casing technology is to apply the natamycin as an external coating. In that case, a food grade coating has to be developed to hold natamycin, and anchor it to the casing wall. However, natamycin is highly insoluble in most of the food grade solvents, including water. So only a minimal amount of natamycin could be incorporated into the casing.

On the other hand, use of resins like of polyamine-polyamide-epichlorohydrin resins, widely used in the casing industry, is not a suitable solution since they require heat to cure, which will decompose the natamycin, and its presence may slow down the drying process of sausage.

Furthermore, in order to obtain a feasible commercial product, the antifungal properties have to remain active against a wide variety of species of mold and yeast for months after the manufacturing date.

The present invention represents a new approach to attach large quantities of the natamycin to the casing, and protect it from degradation by external factors.

### BRIEF DESCRIPTION OF THE FIGURES

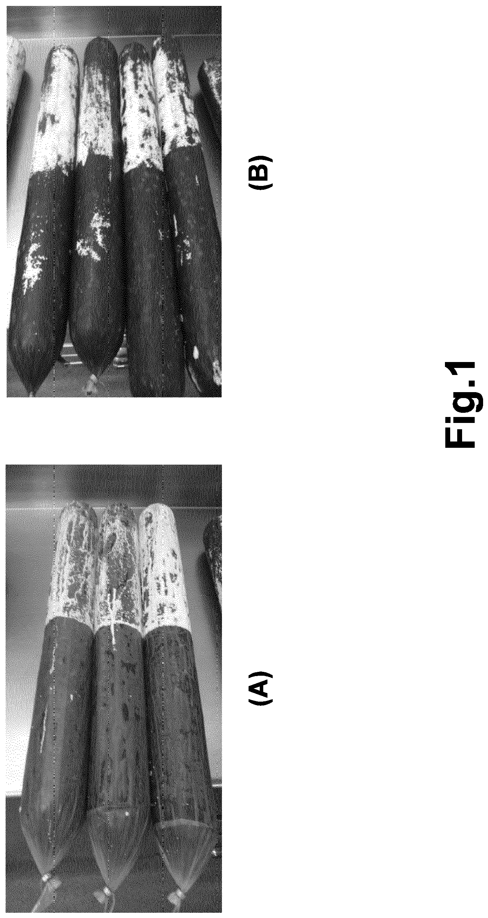

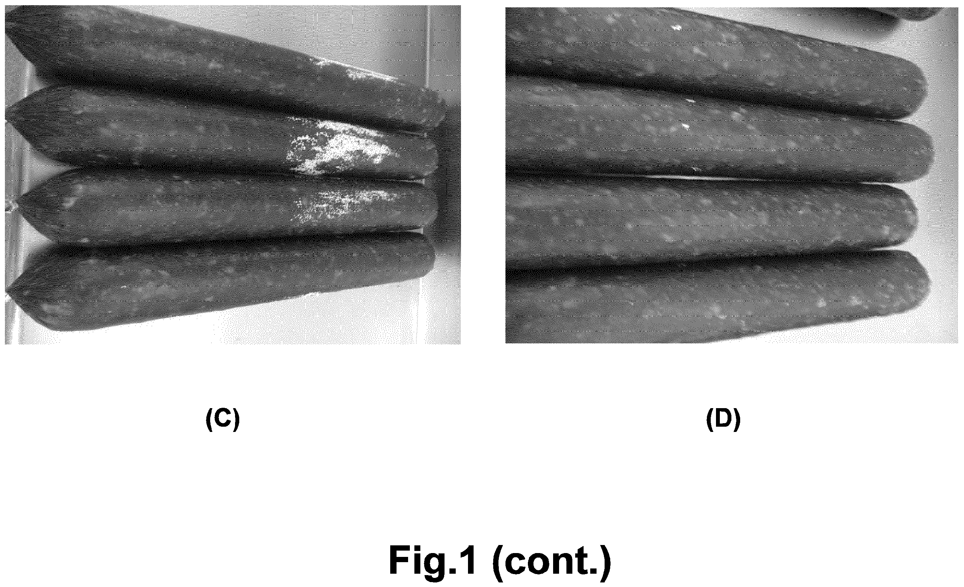

**Figure 1****:** shows the aspect, after two weeks in a curing chamber, of several sausages subjected half of their length to soaking into a water suspension of *Penicilium candidum* containing 10⁶ UFC/gr. The figures shows the aspect of (A) control casing without any natamycin treatment; (B) a casing treated with a water-based composition containing 2% hydroxypropylcelullose and 0.3% Natamycin (composition A); (C) a casing treated with a water-based composition containing 2% Hydroxypropylcellulose, 0.5% linoleic acid, 0.3% Natamycin (composition B); and a casing treated with a water-based composition containing 4% Casein, 1% Beeswax, 3% Carnauba wax, 0.3% Natamycin (composition C). Figures 1 B and C represents the casing according to the invention and clearly show the antifungal effect thereof.

### DESCRIPTION OF THE INVENTION

The present invention is based on a new approach for attaching larger quantities of natamycin to the food casing, which also proved to enhance the antifungal effect of the natamycin. In particular, the present invention is based in the development of a water-based composition comprising at least a) film-forming agent, b) a lipid and c) natamycin which prevents mold and yeast growth without increasing the drying time of the sausage.

It was surprisingly found that this water-based composition, enhance the effect of the natamycin, and preserves the antifungal properties of the antifungal casing for long periods of time after their manufacture.

Therefore, a first aspect of the invention is a food casing with antifungal properties characterized in that it is coated at least in its external surface with a water-based composition comprising at least, a film-forming agent, a lipid, and natamycin.

The food casing may be of any type such as natural casings, collagen casings, cellulose casing, fibrous casing, or polymeric casings. However, in a very preferred embodiment the casing is fibrous casings suitable for the preparation of dry or semidry sausages.

Although not wishing to be bound to any theory, the inventor believes that the film-forming agent and the lipid not only allow anchoring large amounts of natamycin to the casing, which had not been achieved to date, but also increase the availability of the natamycin on the casing surface, as a result of increasing the content of natamycin in the water-based composition. The antifungal casing of the invention has shown not to increase the drying time of the sausage.

The first essential element of the water-based composition applied to the casing is the film-forming agent.

The film-forming agent can be in a percentage by weight of 0.01 to 15% of the total composition, more preferably 0.5 to 8%, and even more preferably from 1 to 5%. The percentage of the film-forming agent is directly related with the amount of the natamycin on the casing.

The amount of the film-forming agent should be established on the basis of the type of film-forming agent and on the contamination of each particular drying chamber.

The film-forming agent can be one of the following: proteins and/or derivatives; polysaccharides and/or derivatives; natural or synthetic resins and/or derivatives; water soluble polymers, or polymers dispersions and/or derivatives or mixtures thereof.

In a preferred embodiment of the invention, the film-forming agent is a protein selected from the following: soy protein, whey protein, pea protein, zein, collagen, casein, gelatin, gluten, keratin, albumins, ovalbumin, bovine serum albumin, derivatives or mixtures thereof; or a polysaccharide from the followings: agar, alginate, carrageenaans, cellulose, carboxymethylcelullose, hydroxypropylcelullose, hydroxyproylmethylcelullose, chitosan, gums, pectin, starch, dextrins or derivatives or mixtures thereof, or a water soluble polymer selected from the following: polyolefins, polyvinyl alcohol, polyvinyl acetate, polyvinyl pyrrolidone, derivatives or mixtures thereof; or a polymer dispersion selected from the following: polyvinyl acetate dispersions stabilized by polyvinyl alcohol, polyvinyl acetate ethylene copolymer stabilized by polyvinyl alcohol, styrene acrylics, vinyl acrylics, styrene butadiene latex, or polyisoprene.

Another component of the water-based composition is the lipid. The lipid may be present in the water-based composition in a percentage by weight of 0.05 to 15% of the total composition, more preferably from 0.5 to 10%, and even more preferably from 1 to 6%. As in the case of the film-forming agent, the lipid percentage has to be sufficient to hold the natamycin to the casing wall. Therefore, the amount of lipid is close connection with the amount of the natamycin in the casing. In turn, the amount of Natamycin and other antifungals will depend on the level of contamination of the drying room where the casing will be stored. Therefore, for more demanding situations the amount of lipid and/or of the film-forming agent must be increased in parallel to the amount of natamycin used.

The lipid can be one of the following: fats, oils, fatty acids, waxes, monoglycerides, diglycerides, triglycerides, derivatives or mixtures thereof.

In a preferred embodiment of the invention the lipid is a natural or synthetic wax, and/or a fatty acid or fatty acid derivative.

In a more preferred embodiment of the invention, the fat is a wax or mixture of them selected from the following: animal waxes such as Beeswax, Chinese wax, Lanolin, Shellac wax, Spermaceti; vegetable waxes such as Bayberry wax, Candelilla wax, Carnauba wax, Castor wax, Esparto wax, Japan wax, Ouricury wax, Rice bran wax, Soy wax, Tallow Tree wax; mineral waxes such as Ceresin waxes, Montan wax, Ozocerite, Peat waxes; or petroleum waxes such Paraffin wax, Microcrystalline wax or any mixture thereof.

The third essential element of the water-based composition applied on the food casings is the natamycin. According to Brik. et al, ["Natamycin" Analytical Profiles of Drug Substances 10, 513-561 (1981)] the solubility of natamycin in water at neutral pH level is 30 ppm (0.003%), while the content of natamycin in the water-based composition of the invention can raised up to 50000 ppm (5%).

Natamycin can be present in a wide range in the water-based composition, depending on the mold and/or yeast population of the drying facility; however it is preferably present in the water-based composition in a percentage of 0.001 to 5% by weight. According to Stark et al, ["Permitted preservatives-Natamycin". Encyclopedia of Food Microbiology (Academic Press, ed. Robinson et. al) 1999 vol. 3:1776-1781] natamycin is effective against most and yeast species at the level of 10 ppm (0.001%). The higher limit will depend on the spore contamination of the drying chamber. The value of 5% is considered enough to produce a full inhibition on an industrial drying chamber with high contamination levels, but even higher concentrations might be needed in some cases. In the preferred embodiment of the invention, the natamycin quantity range is 0.01 to 1% and, even more preferred, between 0.1 to 0.5% of the total composition.

Furthermore, other antifungals may be used in combination with natamycin. The water-based composition may also comprise one or more of the following: sorbic acid, or its salts; benzoic acid or its salts; parabens, propionates or its salts; essential oils or mixture of essential oils like, but not limited to, clove bud, eucalyptus, peppermint, rosemary, lemongrass, lavender, castor, neem oil, or natural extracts from, but not limited to cinnamon, oregano, thyme, chamomile, citrus fruits, propolis, capsaicin; organic saturated short and medium chain acids, and organic unsaturated short and medium chain acids like, but not limited to undecylenic acid, 9-decenoic acid, 8-nonenoic acid. The concentration of these optional antifungals in the water-based composition can vary depending on the antifungal strength of each one but they are preferably within a range of 0-15% by weight in the composition.

The water-based composition can also optionally comprise other additives, such as, plasticizers and/or surfactants or emulsifiers.

Plasticizers are preferably polyols such as glycerol, propylene glycol, sorbitol or xylitol. They can be present in the water-based composition in a percentage of 0 to 15% by weight of the total composition. In a preferred embodiment the plasticizer used is glycerol.

Natural or artificial surfactants or emulsifiers may be present. For example: fatty acids and/or fatty acids salts, sodium lauryl sulphate, dioctyl sulphosuccinate, calcium chloride, or commercial brands are Tween, Span, Brij, and Myrj may be present in a percentage of 0 to 5% by weight of the total composition.

The food casing of the present invention can be designed to be a regular casing or a ready-to-stuff casing, the latter meaning that no soaking in water is needed prior to stuffing. The antifungal casing can be shirred into sticks of the desired length or stay in a reel.

For being a ready-to-stuff casing, the food casings of the invention preferably contain 20-60% more water than regular casings. The desired add-on rate (percentage of casing weight increase) can be achieved during the application of the water-based composition or by internal moisturization. Such an internal moisturization is preferably carried out by application of a diluted solution of weak acid between 0.1 to 5% w/w of acid in water in the internal surface of the casing, more preferably between 0.5 to 3% citric acid solutions.

Another aspect of the invention is related to a method for preparing the food casing with antifungal properties of the invention comprising:
a) manufacturing the casing tube,
b) preparing water-based composition by mixing at least, a film-forming agent, a fat, and natamycin,
c) applying the water-based composition of b) at least on the external surface of the casing.

The process used for the manufacture of the casing (step a) will depend on the nature of the casing, that is, depending on whether the casing is a collagen casing, a cellulose casing, a fibrous casing or polymeric casings. However, process for the manufacture of these type of casings are generally well known in the art.

The preferred embodiment of the invention involves the manufacture of a fibrous casing. The fibrous casing is manufactured using the viscose process. A paper web made of hemp fibers is formed into a tube, which is impregnated internally and externally with viscose. The viscose solution may also contain pigments or other additives. The tube is then introduced in an acidic bath which coagulates the viscose, and chemically converts the viscose into cellulose. The tube is washed to eliminate by-products and later treated with a plasticizer. Finally, the tube is dried, flatted and wound into a reel.

For the preparation of the water-based composition (step b), the components are admixed.

Depending on the selection of the film-forming agent and fat, the procedure of admixing may slightly vary but such variations are within the capabilities of a skilled person.

First, the film-forming agent is mixed into water. For example, in the case of a cellulose derivative, which is very soluble in water, only it is necessary to mix using for instance a high speed mixer. In the case of protein, the pH will most likely need to be adjusted which is within the normal capabilities of a skilled person.

Then, the lipid is preferably slowly mixed into the water phase. If the lipid is solid, it should be melted beforehand. The mixture can be stirred using a high speed mixer. Once the mixture is complete, it must be preferably cooled down. Finally, the natamycin is preferably slowly added to the mixture and stirred.

In step c), the water-based composition is then applied at least on the external surface surface of the casing prepared in a). The impregnation of at least the external surface of the casing can be done by any suitable method such as, for instance, soaking, spraying or printing of the water-based antifungal coating composition.

The soaking method is preferred. The flat casing is passed through a dip tank containing the water-based composition. Since in a preferred embodiment of the invention the casing is a ready-to-stuff casing with high moisture content, the final weight increase may vary depending on the product requirements, for example, between 20-60%.

The desired water add-on [percentage of casing weight increase] could be obtained during the application of the water-based composition only (step c), or additionally, including a previous step of internal moisturization of the casing.

Such optional step of moisturization of the internal surface of the casing prior to step c), is, in a preferred embodiment, carried out by applying a weak acid water solution, or a weak acid solution with a plasticizer. For instance, moisturization can be achieved by applying a diluted solution of a weak acid between 0.1 to 5% w/w of acid in water. In a preferred embodiment the moisturizing solution is a diluted solution of citric acid, an even more preferably between 0.5 to 3% citric acid solution. This moisturizing step can lead to an increase of the casing weight that may vary depending on the product requirements, typically between 15-40 %.

Depending on the applications the antifungal casing could be additionally impregnated with the water-based composition, or a dilution of the water-based composition, in the internal face of the casing.

After the application of the water-based composition, the casing sits for a few hours to consolidate the coating, and optionally the flat casing coated with the water-based composition can be shirred with or without lubrication, and converted into sticks of the desired length.

The resulting antifungal casing can hold at least 1 milligram of natamycin per decimeter square of casing, and it is highly effective against a wide variety of mold and yeast species.

The antifungal casing of this invention remains active for at least 4 months after the manufacturing date, and allows drying the sausage in a similar period of time than a standard casing.

As a final object, the invention is also directed to a meat product comprising a food casing with antifungal properties as previously disclosed stuffed with meat or a meat emulsion. In a preferred embodiment, the meat products are sausages and more particularly dry or semidry sausages.

The invention will be described in more detail by means of the following examples. The description below discloses some embodiments and examples of the invention in such detail that a person skilled in the art is able to utilize the invention based on the disclosure. Not all the steps of the embodiments are disclosed in detail, as many of them will be obvious for a person skilled in the art.

### EXAMPLES

### EXAMPLE 1: Effect of the casing of the invention against molds and yeast

A Double Viscose Layer (DVL) Viscofan Fibrous Clear Securex Code 4 S flat casing was coated passing through a dip tank containing with one the following water-based compositions:
- Composition A: 2% Hydroxypropylcelullose, 0.3% Natamycin
- Composition B: 2% Hydroxypropylcellulose, 0.5% linoleic acid, 0.3% Natamycin
- Composition C: 4% Casein, 1% Beeswax, 3% Carnauba wax, 0.3% Natamycin.

Composition B and C are part of the invention whereas A is a control.

The add-on rate [percentage of casing weight increase after the impregnation] was 40%. The antifungal casing went through a shirring machine, which converted the flat casing into sticks of the desired length.

The antimold casing was stuffed with raw sausage meat fermented for 24 hours, and then dried for 2.5 weeks in an industrial drying room naturally contaminated with *Penicillium commune* (mold), *Cladosporium sphaerospermum* (mold), and *Debaromyces hansenii* (yeast).

The stuffed antifungal casing of this invention was compared with:
- Control casing (control 01): fibrous casing not treated with any antifungal coating or compounds and stuffed with the same meat emulsion.
- Treated control casing (control 02): same stuffed fibrous casing as "Control 01", which was soaked with a 0.3% antifungal solution of natamycin in water after being stuffed with the same meat emulsion as the antifungal casing and the control casing 01.

Results are summarized in the table 1.

**Table 1**

| | **Percentage of casing surface contaminated with mold/yeast at the end of the test** |
|---|---|
| Control casing 01 | 90% |
| Treated control casing 02 | 70% |
| Casing coated with composition A | 65% |
| Casing coated with composition B | 15% |
| Casing coated with composition C | 2% |

At the end of the test, a 90% of the surface of the control casing (control 01) was contaminated, mainly with *D. hansenii.* The treated control casing (control 02) was contaminated in 70% of its surface. Composition A did not show any significant improvement compared with the treated control casing (control 02). Even though composition A, B and C, as well as the treated control (02) were impregnated with the same percentage of natamycin (0.3%), the compositions based on the invention (composition B and composition C), showed an almost complete inhibition of *Debaromyces hansenii,* and a full inhibition of *Penicillium commune,* and *Cladosporium sphaerospermum.*

### EXAMPLE 2: Drying rate of the casing of the invention

The antifungal casings described in Example 1 were tested against *Penicilium candidum,* and the water loss of the antifungal casing was monitored, and compared with a control of the same fibrous casing without any antifungal treatment (control 01).

The antifungal casing and the control were stuffed with raw sausage meat. The weight of each stuffed casing (raw meat + casing) was recorded.

Only half of each stuffed casing was soaked into a water suspension of *Penicilium candidum* from Danisco containing 10⁶ UFC/gr.

The stuffed casings (antifungal and control) were checked weekly, and their weights were recorded. After two weeks, the drying was considered finished.

As can be observed in figure 1, the full surface of the antimold casings of this invention was substantially clean, with the exception of a few isolated small colonies (figure 1 B and C). However, the area from the control casing, which was soaked into the mold culture, was fully contaminated with *Penicilium candidum* (see figure 1 A) .

The results in Table 2 also showed that the antimold casing of the invention and the control lost a similar amount of the water in the same period of time. This proves that the coating of the invention does not impede or slow down the drying cycle of the sausage.

**Table 2**

| | **ID** | **Weight/ Kg After stuffing** | **Weight/g after 1 week** | **% water loss After one week** | **Weightlg At the end of the test** | **% water loss At the end of the test** | **Bottom middle area of the stuffed casing Percentage of mold coverage** |
|---|---|---|---|---|---|---|---|
| **Control 01 standard casing** | 1 | 3115 | 2545 | 18.3 | 2190 | 29.7 | 99 |
| | 2 | 3100 | 2535 | 18.2 | 2225 | 28.2 | 99 |
| | 3 | 3005 | 2520 | 16.1 | 2190 | 27.1 | 99 |
| | 4 | 3100 | 2525 | 18.5 | NA | NA | NA |
| **Composition A** | 5 | 2850 | 2310 | 18,9 | 2015 | 29,3 | 80 |
| | 6 | 2945 | 2370 | 19,5 | 2075 | 29,5 | 60 |
| | 7 | 2860 | 2315 | 19,1 | 1995 | 30,2 | 75 |
| | 8 | 2930 | 2390 | 18,4 | 2055 | 29,9 | 80 |
| **Composition B** | 9 | 2865 | 2335 | 18,5 | 2035 | 29,0 | 2 |
| | 10 | 2915 | 2405 | 17,5 | 2095 | 28,1 | 5 |
| | 11 | 2860 | 2360 | 17,5 | 2030 | 29,0 | 1 |
| | 12 | 2950 | 2425 | 17,8 | 2085 | 29,3 | 0 |
| **Composition C** | 13 | 2860 | 2325 | 18.7 | 2005 | 29.9 | 0.1 |
| | 14 | 2935 | 2430 | 17.2 | 2070 | 29.5 | 0.1 |
| | 15 | 2935 | 2440 | 16.9 | 2075 | 29.3 | 0 |
| | 16 | 2870 | 2375 | 17.2 | 2025 | 29.4 | 0 |

### EXAMPLE 3: Duration of the antifungal effect of the casing

The shelf life of an antifungal casing according to the composition C was monitored for several months. The casing was stored in a dark room, at room temperature, and atmospheric conditions.

The natamycin was extracted from the Antifungal casing using methanol, and analyzed with an UV-Vis spectrophometer. Natamycin produces a spectrum with 3 peaks at 290, 303 and 318nm. A calibration curve correlating the absorbance with the natamycin concentration was created beforehand.

| | After manufacturing | After 2 months | After 4 months |
|---|---|---|---|
| Natamycin mg/dm² on the antifungal casing of the invention | 0.95 + 0.09 | 0.89 + 0.04 | 0.88 + 0.06 |

No significant differences were observed in the concentration of natamycin in the casing overtime. This confirms that the antifungal casing of the invention has a shelf-life of at least 4 months.

The scope of protection of the current invention is defined by the appended claims.

## Claims

1. A food casing with antifungal properties **characterized in that** it is coated at least in its external surface with a water-based composition comprising at least, a film forming agent, a lipid and natamycin.

2. A food casing according to any of the previous claims wherein the water-based composition comprises the film-forming agent in a percentage by weight of 0.01 to 15%.

3. A food casing according to any of the previous claims wherein the film-forming agent is selected from proteins and/or derivatives; polysaccharides and/or derivatives; natural or synthetic resins and/or derivatives, water soluble polymers or polymers dispersions and/or derivatives or mixtures thereof.

4. A food casing according to any of the previous claims wherein the film-forming agent is a protein selected from soy protein, whey protein, pea protein, zein, collagen, casein, gelatin, gluten, keratin, albumins, ovalbumin, bovine serum albumin, derivatives or mixtures thereof; or a polysaccharide selected from agar, alginate, carrageenans, cellulose, carboxymethylcelullose, hydroxypropylcelullose, hydroxyproylmethylcelullose, chitosan, gums, pectin, starch, dextrins or derivatives or mixtures thereof; or a water soluble polymer selected from polyolefins, polyvinyl alcohol, polyvinyl acetate, polyvinyl pyrrolidone, derivatives or mixtures thereof; or a polymer dispersion selected from polyvinyl acetate dispersions stabilized by polyvinyl alcohol, polyvinyl acetate ethylene copolymer stabilized by polyvinyl alcohol, styrene acrylics, vinyl acrylics, styrene butadiene latex, or polyisoprene.

5. A food casing according to claim 1 wherein the water-based composition comprises the lipid in a percentage by weight of 0.05 to 15%.

6. A food casing according to any of the previous claims where the lipid is selected from a fats, oils, fatty acids, waxes, monoglycerides, diglycerides, triglycerides or derivatives or mixtures thereof.

7. A food casing according to any of the previous claims where the lipid is a wax selected from Beeswax, Chinese wax, Lanolin, Shellac wax, Spermaceti, Bayberry wax, Candelilla wax, Carnauba wax, Castor wax, Esparto wax, Japan wax, Ouricury wax, Rice bran wax, Soy wax, Tallow tree wax, Ceresin waxes, Montan wax, Ozocerite, Peat waxes, Paraffin wax, Microcrystalline wax or mixtures thereof.

8. A food casing according to any of the previous claims wherein the water based composition comprises natamycin in a percentage by weight of 0.001 to 5%.

9. A food casing according to any of the previous claims which is a ready-to-stuff casing.

10. A food casing according to any of the previous claims which is a fibrous casing.

11. A food casing according to any of the previous claims where the water-based composition additionally comprises other additives selected from plasticizers, surfactants and/or emulsifiers.

12. A food casing according to any of the previous claims additionally comprising a further antifungal or mixtures of antifungals.

13. A food casing according to any of the previous claims additionally comprising fatty acids and/or fatty acid salts.

14. A method for preparing a food casing according to claim 1 comprising:
a. manufacturing the casing tube,
b. preparing water-based composition by mixing at least, a film-forming agent, a lipid and natamycin,
c. applying the water-based composition of b) at least on the external surface of the casing.

15. A method according to claim 14 where optionally prior to step c) the internal surface of the casing is moisturized, preferably with a water solution of a weak acid.

16. A method according to any of claims 14 or 15 where the water-based composition of step c) is applied by soaking, spraying or printing.

17. A meat product comprising a food casing according to any of claim 1-12 stuffed with meat or a meat emulsion.

## Patentansprüche

1. Lebensmittelhülle mit antimykotischen Eigenschaften, **dadurch gekennzeichnet, dass** sie auf ihrer äußeren Oberfläche mit einer wasserbasierenden Zusammensetzung beschichtet ist, die wenigstens ein filmbildendes Mittel, ein Lipid und Natamycin umfasst.

2. Lebensmittelhülle nach einem der vorangehenden Ansprüche, wobei die wasserbasierende Zusammensetzung das filmbildende Mittel in einem gewichtsprozentualen Anteil von 0,01 bis 15% umfasst.

3. Lebensmittelhülle nach einem der vorangehenden Ansprüche, wobei das filmbildende Mittel ausgewählt ist aus Proteinen und/oder Derivaten, Polysacchariden und/oder Derivaten, natürlichen oder synthetischen Harzen und/oder Derivaten, wasserlöslichen Polymeren oder Polymerdispersionen und/oder Derivaten oder Mischungen davon.

4. Lebensmittelhülle nach einem der vorangehenden Ansprüche, wobei das filmbildende Mittel ein Protein ist, ausgewählt aus Sojaprotein, Molkeprotein, Erbsenprotein, Zein, Kollagen, Kasein, Gelatine, Gluten, Keratin, Albuminen, Ovalbumin, bovinem Serumalbumin, Derivaten oder Mischungen davon; oder ein Polysaccharid, ausgewählt aus Agar, Alginat, Carrageenanen, Cellulose, Carboxymethylcellulose, Hydroxypropylcellulose, Hydroxyproylmethylcellulose, Chitosan, Gummis, Pektin, Stärke, Dextrinen oder Derivaten oder Mischungen davon; oder ein wasserlösliches Polymer, ausgewählt aus Polyolefinen, Polyvinylalkohol, Polyvinylacetat, Polyvinylpyrrolidon, Derivaten oder Mischungen davon; oder eine Polymerdispersion, ausgewählt aus Polyvinylacetat-Dispersionen, die durch Polyvinylalkohol stabilisiert sind, Polyvinylacetatethylen-Copolymer, stabilisiert durch Polyvinylalkohol, Styrol-Acryle, Vinylacryle, StyrolButadien-Latex oder Polyisopren.

5. Lebensmittelhülle nach Anspruch 1, wobei die wasserbasierende Zusammensetzung das Lipid in einem gewichtsprozentualen Anteil von 0,05 bis 15 % umfasst.

6. Lebensmittelhülle nach einem der vorangehenden Ansprüche, wobei das Lipid aus Fetten, Ölen, Fettsäuren, Wachsen, Monoglyceriden, Diglyceriden, Triglyceriden oder Derivaten oder Mischungen davon ausgewählt ist.

7. Lebensmittelhülle nach einem der vorangehenden Ansprüche, wobei das Lipid ein Wachs ist, ausgewählt aus Bienenwachs, Chinawachs, Lanolin, Schellackwachs, Spermaceti, Bayberrywachs, Candelillawachs, Carnaubawachs, Rhizinuswachs, Esparto-Wachs, Japanwachs, Quecksilberwachs, Reiskleiewachs, Sojawachs, Talgbaumwachs, Ceresin-Wachse, Montanwachs, Ozokerit, Torfwachse, Paraffinwachs, mikrokristallinem Wachs oder Mischungen davon.

8. Lebensmittelhülle nach einem der vorangehenden Ansprüche, wobei die wasserbasierende Zusammensetzung Natamycin in einem gewichtsprozentualen Anteil von 0,001 bis 5 % umfasst.

9. Lebensmittelhülle nach einem der vorangehenden Ansprüche, die eine stopffertige Hülle ist.

10. Lebensmittelhülle nach einem der vorangehenden Ansprüche, die eine faserartige Hülle ist.

11. Lebensmittelhülle nach einem der vorangehenden Ansprüche, wobei die wasserbasierende Zusammensetzung zusätzlich andere Zusatzstoffe umfasst, ausgewählt aus Weichmachern, oberflächenaktiven Mitteln und/oder Emulgatoren.

12. Lebensmittelhülle nach einem der vorangehenden Ansprüche, die zusätzlich ein weiteres antimykotisches Mittel oder Mischungen von antimykotischen Mitteln umfasst.

13. Lebensmittelhülle nach einem der vorangehenden Ansprüche, welche zusätzlich Fettsäuren und/oder Fettsäuresalze umfasst.

14. Verfahren zum Herstellen einer Lebensmittelhülle nach Anspruch 1, umfassend:
a) Herstellen des Hüllenschlauchs,
b) Zubereitung einer wasserbasierenden Zusammensetzung durch Mischen von wenigstens einem filmbildenden Mittel, einem Lipid und Natamycin,
c) Aufbringen der wasserbasierenden Zusammensetzung b) wenigstens auf der äußeren Oberfläche der Hülle.

15. Verfahren nach Anspruch 14, wobei fakultativ vor Schritt c) die interne Oberfläche der Hülle befeuchtet wird, bevorzugt mit einer Wasserlösung einer schwachen Säure.

16. Verfahren nach einem der Ansprüche 14 oder 15, wobei die wasserbasierte Zusammensetzung von Schritt c) durch Einweichen, Sprühen oder Drucken aufgebracht wird.

17. Fleischprodukt, umfassend eine Lebensmittelhülle nach einem der Ansprüche 1-12, welche mit Fleisch oder einer Fleischemulsion gefüllt ist.

## Revendications

1. Boyau alimentaire ayant des propriétés antifongiques **caractérisé en ce qu'**il est enrobé au moins dans sa surface externe avec une composition à base d'eau comprenant au moins, un agent filmogène, un lipide et de la natamycine.

2. Boyau alimentaire selon l'une quelconque des revendications précédentes dans lequel la composition à base d'eau comprend l'agent filmogène en un pourcentage en poids de 0,01 à 15 %.

3. Boyau alimentaire selon l'une quelconque des revendications précédentes dans lequel l'agent filmogène est choisi parmi les protéines et/ou des dérivés ; les polysaccharides et/ou des dérivés ; les résines naturelles ou synthétiques et/ou des dérivés, les polymères hydrosolubles ou les dispersions de polymères et/ou des dérivés ou des mélanges de ceux-ci.

4. Boyau alimentaire selon l'une quelconque des revendications précédentes dans lequel l'agent filmogène est une protéine choisie parmi la protéine de soja, la protéine de lactosérum, la protéine de pois, la zéine, le collagène, la caséine, la gélatine, le gluten, la kératine, les albumines, l'ovalbumine, l'albumine de sérum bovin, des dérivés ou des mélanges de ceux-ci ; ou un polysaccharide choisi parmi l'agar-agar, l'alginate, les carraghénanes, la cellulose, le carboxyméthylcellulose, l'hydroxypropylcellulose, l'hydroxyproylméthylcellulose, le chitosane, les gommes, la pectine, l'amidon, les dextrines ou des dérivés ou des mélanges de ceux-ci ; ou un polymère hydrosoluble choisi parmi les polyoléfines, un alcool polyvinylique, un acétate de polyvinyle, une polyvinylpyrrolidone, des dérivés ou des mélanges de ceux-ci ; ou une dispersion de polymère choisie parmi les dispersions d'acétate de polyvinyle stabilisées par de l'alcool polyvinylique, un copolymère d'éthylène et d'acétate de polyvinyle stabilisé par de l'alcool polyvinylique, des acryliques styrène, des acryliques vinyliques, un latex styrène butadiène ou un polyisoprène.

5. Boyau alimentaire selon la revendication 1 dans lequel la composition à base d'eau comprend le lipide en un pourcentage en poids de 0,05 à 15 %.

6. Boyau alimentaire selon l'une quelconque des revendications précédentes où le lipide est choisi parmi les graisses, les huiles, les acides gras, les cires, les monoglycérides, les diglycérides, les triglycérides ou des dérivés ou des mélanges de ceux-ci.

7. Boyau alimentaire selon l'une quelconque des revendications précédentes où le lipide est une cire choisie parmi la cire d'abeille, la cire de Chine, la lanoline, la cire de shellac, le spermaceti, la cire de baie de laurier, la cire de candelilla, la cire de carnauba, la cire de ricin, la cire de sparte, la cire du Japon, la cire d'ouricury, la cire de son de riz, la cire de soja, la cire de suif, les cires de cérésine, la cire de lignite, l'ozocérite, les cires de tourbe, la cire de paraffine, la cire microcristalline ou des mélanges de ceux-ci.

8. Boyau alimentaire selon l'une quelconque des revendications précédentes dans lequel la composition à base d'eau comprend de la natamycine en un pourcentage en poids de 0,001 à 5 %.

9. Boyau alimentaire selon l'une quelconque des revendications précédentes qui est un boyau prêt à farcir.

10. Boyau alimentaire selon l'une quelconque des revendications précédentes qui est un boyau fibreux.

11. Boyau alimentaire selon l'une quelconque des revendications précédentes où la composition à base d'eau comprend en outre d'autres additifs choisis parmi les plastifiants, les tensioactifs et/ou les émulsifiants.

12. Boyau alimentaire selon l'une quelconque des revendications précédentes comprenant en outre un autre antifongique ou des mélanges d'antifongiques.

13. Boyau alimentaire selon l'une quelconque des revendications précédentes comprenant en outre des acides gras et/ou des sels d'acides gras.

14. Procédé de préparation d'un boyau alimentaire selon la revendication 1 comprenant :
a. la fabrication du tube de boyau,
b. la préparation d'une composition à base d'eau en mélangeant au moins, un agent filmogène, un lipide et de la natamycine,
c. l'application de la composition à base d'eau de b) au moins sur la surface externe du boyau.

15. Procédé selon la revendication 14 où éventuellement avant l'étape c) la surface interne du boyau est humidifiée, de préférence avec une solution aqueuse d'un acide faible.

16. Procédé selon l'une quelconque des revendications 14 ou 15 où la composition à base d'eau de l'étape c) est appliquée par trempage, pulvérisation ou impression.

17. Produit carné comprenant un boyau alimentaire selon l'une quelconque des revendications 1 à 12 farci avec de la viande ou une émulsion de viande.
